# NEW EUROPEAN PATENT SPECIFICATION

(11) **EP 2 658 541 B2**
(45) Date of publication and mention of the opposition decision: **17.12.2025**
(45) Mention of the grant of the patent: 26.01.2022
(21) Application number: 11799292.5
(22) Date of filing: 22.12.2011
(51) Int. Cl.: A61K 9/08, A61K 31/42, A61P 33/14, A61K 47/18, A61K 47/34, A01N 43/80, A01P 7/04

(54) **TOPICAL LOCALIZED ISOXAZOLINE FORMULATION COMPRISING GLYCOFUROL**
TOPISCH LOKALISIERTE ISOXAZOLINFORMULIERUNG MIT GLYCOFUROL
FORMULATION D'ISOXAZOLINE D'APPLICATION TOPIQUE LOCALE COMPRENANT DU GLYCOFUROL

(30) Priority: 27.12.2010 EP 10197090; 06.01.2011 US 201161430241 P
(43) Date of publication of application: 06.11.2013
(73) Proprietor: Intervet International B.V., 5831 AN Boxmeer (NL)
(72) Inventor: FUCHS, Stefan, 55270 Schwabenheim (DE); HECKEROTH, Anja Regina, 55270 Schwabenheim (DE); MÜLLER, Ramona, 55270 Schwabenheim (DE); WILLIAMS, Heike, 55270 Schwabenheim (DE); ZOLLER, Hartmut, 55270 Schwabenheim (DE)
(74) Representative: Intervet International B.V.
(86) International application number: PCT/EP2011/073830
(87) International publication number: WO 2012/089623

(56) References cited:
- EP-B1- 2 658 542
- WO-A1-2007/018659
- WO-A1-2018/039508
- WO-A2-2009/024541
- WO-A2-2009/024541
- WO-A2-2011/157733
- US-A1- 2010 311 685
- US-A1- 2010 311 685
- OZOE, Y. ET AL: "The antiparasitic isoxazoline A1443 is a potent blocker of insect ligand-gated chloride channels", BIOCHEMICAL AND BIOPHYSICAL RESEARCH COMMUNICATIONS, vol. 391, no. 1, 1 January 2010 (2010-01-01), Amsterdam NL , pages 744 - 749, XP026823770, ISSN: 0006-291X, DOI: 10.1016/j.bbrc.2009.11.131
- TAENZLER JANINA, ET AL: "Efficacy of fluralaner spot-on solution against induced infestations with Rhipicephalus sanguineus on dogs", PARASITES & VECTORS, vol. 9, no. 276, 1 January 2016 (2016-01-01), pages 1 - 5, XP055977232
- BRAVECTO® SPOT-ON (MARKETING AUTHORIZATION
- "Ectoparasites - Drug discovery against moving targets", 1 January 2018, WILEY VCH, ISBN: 978-3-527-34168-9, article XU MING, ET AL: "13 The Discovery of Afoxolaner: A New Ectoparasiticide for Dogs", pages: 259 - 271, XP055977348

## Description

### FIELD OF THE INVENTION

This invention provides topical localized formulations comprising an isoxazoline compound and a pharmaceutically or veterinary acceptable liquid carrier vehicle. This invention also provides a formulation for use in an improved method for controlling, and preventing parasite infestation in animals.

### BACKGROUND OF THE INVENTION

A number of pests and parasites can infest or infect domestic animals such as cattle, horses, pigs, sheep and also companion animals such as cats and dogs. These pests and parasites are of great nuisance to both the animals and their owners.

Ectoparasites such as ticks, mites, lice, flies and fleas irritate the animals and can cause disease, either by themselves, or by carrying vector transmitted pathogens.

New economic methods and compositions for the prevention, treatment and control of parasites in warm- blooded animals are constantly being sought.

A new family of insecticide isoxazoline compounds has been described in various patent applications; for example, in US patent application US 2007/0066617, and International Patent applications WO 2007/079162, WO 2009/002809, WO 2009/024541, WO 2009/003075, WO 2010/070068 and WO 2010/079077.

As these isoxazoline compounds have been originally investigated for their use in the agricultural area it is necessary to identify specific formulations that allow their veterinary use, i.e. safe administration to control parasites in animal effectively.

One known and convenient way of administering an ectoparasiticide compound to an animal is the topical localized administration, e.g. as spot-on or pour-on.

However, prior art formulations and conventional topical localized ectoparasiticide formulations using suggested solvents for isoxazoline compounds have difficulties applying effective amounts of isoxazoline compounds with acceptable cosmetic appearance. Particularly, high volumes of conventional topical localized formulations can result in product run-off and sodden appearances of the fur after administration and high concentration formulations can result in insolubility (crystallization) of the active ingredient, skin irritation as well as undesirable product characteristics, such as poor viscosity, insufficient spreading, poor evaporation and inadequate permeation.

Thus, what is needed in the art, are topical localized formulations and dosage regimens of isoxazoline compounds, which avoid the drawbacks mentioned above.

### SUMMARY OF THE INVENTION

The current invention provides topical localized formulations for the administration of an isoxazoline compound being 4-[5-(3,5-dichlorophenyl)-5-trifluoromethyl-4,5-dihydroisoxazol-3-yl]-2-methyl-*N*-[(2,2,2-trifluoro-ethylcarbamoyl)-methyl]-benzamide that overcome the drawbacks of the prior art. The formulations of the invention deliver effective amounts of the isoxazoline compound after topical localized administration and with acceptable cosmetic appearance.

The current invention is directed to a topical localized formulation for the treatment or prophylaxis of parasite infestation in animals which comprises an effective amount of at least one isoxazoline compound being 4-[5-(3,5-dichlorophenyl)-5-trifluoromethyl-4,5-dihydroisoxazol-3-yl]-2-methyl-*N*-[(2,2,2-trifluoro-ethylcarbamoyl)-methyl]-benzamide.

A topical localized spot-on or pour- on formulation for use in the treatment or prophylaxis of flea and tick infestation in dogs or cats which comprises an effective amount of at least one isoxazoline compound being 4-[5-(3,5-dichlorophenyl)-5-trifluoromethyl-4,5-dihydroisoxazol-3-yl]-2-methyl-*N*-[(2,2,2-trifluoro-ethylcarbamoyl)-methyl]-benzamide, and a veterinary acceptable liquid carrier vehicle wherein the liquid carrier vehicle comprises glycofurol as a solvent, and a co-solvent selected from the group consisting of acetone, acetonitrile, benzyl alcohol, butyl diglycol, dimethylacetamide, dimethylsulfoxide, dimethylformamide, N,N-diethyl-3-methylbenzamide , dipropylene glycol n-butyl ether, ethyl alcohol, isopropanol, methanol, phenylethyl alcohol, isopropanol, ethylene glycol monoethyl ether, ethylene glycol monomethyl ether, monomethylaceamide, dipropylene glycol monomethyl ether, liquid polyoxyethylene glycols, propylene glycol, N-methylpyrrolidone, 2-pyrrolidone, limonene, eucalyptol, diethylene glycol monoethyl ether, ethylene glycol, diethyl phthalate, polyethoxylated castor oil, methyl ethyl ketone, ethyl-L-lactate, and a mixture of at least two of these co-solvents; the formulation comprises 20 - 35% w/v of the isoxazoline compound in an application volume of 0.3 to 6 ml per animal; with long acting efficacy against ticks and fleas, so that the administration is carried out monthly, every 2 months, 3 months, 4 months, 5 months or 6 months.

In one embodiment the composition comprises additionally an effective amount of a macrocyclic lactone compound selected from ivermectin, moxidectin, milbemycin oxime, selamectin, emamectin, latidectin and lepimectin or a salt thereof and/ or an insect growth regulator compound selected from fenoxycarb, lufenuron, diflubenzuron, novaluron, triflumuron, fluazuron, cyromazine, methoprene and pyriproxyfen.

These and other embodiments are disclosed and encompassed by the following Detailed Description.

### BRIEF DESCRIPTION OF THE DRAWINGS

Figure 1: Plasma concentration of compound A after spot-on administration of formulations F, D and G to Beagle dogs
Figure 2: Plasma concentration of Compound A after spot-on administration of formulation H to Beagle dogs
Figure 3 Compound A and moxidectin plasma concentration after spot-on administration of formulation G to Beagle dogs

### DETAILED DESCRIPTION OF THE INVENTION

The topical localized formulation according to the invention comprises an isoxazoline compoundwhich is 4-[5-(3,5-Dichlorophenyl)-5-trifluoromethyl-4,5-dihydroisoxazol-3-yl]-2-methyl-*N*-[(2,2,2-trifluoro-ethylcarbamoyl)-methyl]-benzamide (CAS RN [864731-61-3]).

In another embodiment the compound is (Z)-4-[5-(3,5-Dichlorophenyl)-5-trifluoromethyl-4,5-dihydroisoxazol-3-yl]-N-[(methoxyimino)methyl]-2-methylbenzamide (CAS RN [928789-76-8]).

According to the invention the isoxazoline compound is

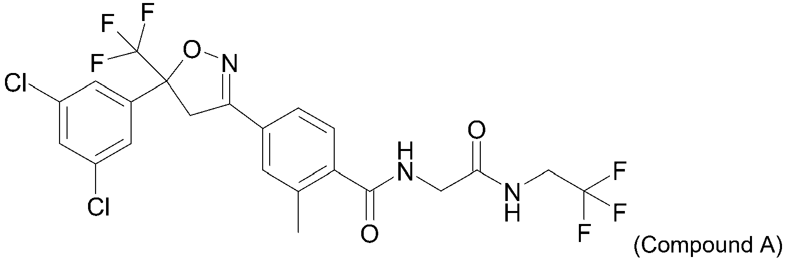

Isoxazoline compounds are known in the art and these compounds and their use as parasiticide are described, for example, in US patent application No. US 2007/0066617, and International Patent applications WO 2007/079162, WO 2009/002809, WO 2009/024541, WO 2009/003075, WO 2010/070068 , WO 2010/079077, WO 2011/075591 and WO 2011/ 124998, the disclosures of which, as well as the references cited herein, are incorporated by reference. This class of compounds is known to possess excellent activity against ectoparasites such as ticks and fleas.

The isoxazoline compounds may exist in various isomeric forms. A reference to an isoxazoline compound always includes all possible isomeric forms of such compound.

Unless otherwise stated, a compound structure that does not indicate a particular conformation is intended to encompass compositions of all the possible conformational isomers of the compound, as well as compositions comprising fewer than all the possible conformational isomers. In some embodiments, the compound is a chiral compound. In some embodiments, the compound is a non-chiral compound.

Isoxazoline compounds can be prepared according to one or other of the processes described in Patent Applications US 2007/0066617, WO 2007/079162, WO 2009/002809, WO 2010/070068 and WO 2010/079077 or any other process coming within the competence of a person skilled in the art who is an expert in chemical synthesis. For the chemical preparation of the products of the invention, a person skilled in the art is regarded as having at his disposal, inter alia, the entire contents of "Chemical Abstracts" and of the documents which are cited therein.

The formulations according to the invention are effective for long durations of time in the treatment of ectoparasites of mammals and, in particular, of fleas and ticks in small mammals such as dogs and cats. Advantageously, the formulations of the invention retain the desired physical characteristics over time, without loss of potency of the active. Further, the formulations of the invention exhibit sufficient viscosity, which allows for the retention of said composition when administered topically to an animal's skin or hair.

Furthermore the formulations of the current invention have favorable product characteristics i.e. they are stable and are cosmetically acceptable.

Cosmetic acceptability includes the (absence of) smell of hair and skin, wetness of the hair and skin of the application site, the overall appearance of the dogs' coat, particularly signs such as dryness, wiry look, brittleness, dullness, hair loss and the appearance of residue of the hair in the proximity of the administration site.

Such cosmetic acceptability is very important for products for topical localized administration to companion animals like dogs and cats, because the pet owner would not accept long lasting changes in the appearance of the fur of their pet following the administration.

With the formulations according to the current invention it was possible to identify topical localized formulations that allow the administration of isoxazoline compounds for a long acting efficacy against ticks and fleas while being cosmetically acceptable.

Topical localized formulations are understood to refer to a ready-to-use formulation in form of a spot-on or pour-on formulation. The expression spot-on or pour-on method is understood to refer to a ready-to-use concentrate intended to be applied topically and locally on the animal This sort of formulation is intended to be applied directly to a relatively small area of the animal, preferably on the animal's back and breech or at one or several points along the line of the back and breech.

Spot-on administration is a topical localized administration of a concentrated solution, suspension, microemulsion or emulsion for intermittent application to a spot on the animal, generally between the two shoulders in 1, 2, 3, 4, or 5 locations (spots), if more than one spot preferably down the back of the animal. Alternatively the product is administered by administering a line.

The pour-on formulation is typically applied by pouring in one or several lines or in a spot-on the dorsal midline (back) or shoulder of an animal. More typically, the formulation is applied by pouring it along the back of the animal, following the spine. The pour-on formulations of this invention can be in the form of a liquid, emulsion, foam, paste, aerosol, ointment, salve or gel. Typically, the pour-on formulation is liquid.

The formulation can also be applied to the animal by other conventional methods, including wiping an impregnated material over at least a small area of the animal, or applying it using a commercially available applicator, by means of a syringe, by spraying or by using a spray race.

A pour-on or spot-on formulation generally can advantageously comprise the isoxazoline compound in a proportion of about 20 to 35%, 25 to 30% about 20%, 25%, 28%, 30%, 33%, (percentages as weight by volume=W/V).

The topical localized formulation allows or facilitates the isoxazoline compound to penetrate the skin and act on other body parts (e.g., the entire body). Such a pour-on or spot-on formulation can be prepared by dissolving, suspending, or emulsifying the isoxazoline in a suitable veterinarily acceptable carrier.

The topical localized formulation comprises a carrier comprising glycofurol and at least one additional veterinary acceptable co-solvent as described in this specification.

Glycofurol is a well known chemical compound. Various syntheses for the preparation thereof are well-known to the art. Glycofurol (CAS No. 9004--76-6 or 31692-85-0), also known as tetrahydrofurfuryl alcohol polyethylene glycol ether or α-(tetrahydrofuranyl)-ω- hydroxypoly(oxy-1,2-ethanediyl) has an average molecular weight of ca. 190; a b.p. of from ca. 80 - 100 °C., a density of ca. 1.070-1.090 g/cm3 (at 20 °C); a hydroxyl value of ca. 300-400; a refractive index of ca. 1.4545 (sodium D line, 589 mm) (at 40 °C); and a viscosity of ca. 8-18 mPa·s (at 20 °C). (c.f. "Handbook of Pharmaceutical Excipients, published by American Pharmaceutical Association/ The Pharmaceutical Society of Great Britain (1986), p. 127 and Fiedler, "Lexikon der Hilfstoffe", 3rd edition (1989), p. 577.)

The precise properties of glycofurol vary according to composition and purity. Thus lower quality grades contain significant amounts of tetrahydrofurfuryl alcohol and other impurities. Synonym names for glycofurol are: Glycofurol 75; tetraglycol; Poly(oxy-1,2-ethanediyl), α-(tetrahydrofuranyl)-ω-hydroxy- (9CI). Tetraglycol is also used as a synonym for a different chemical compound, tetrahydrofurfuryl alcohol.

A pour-on or spot-on formulation generally can advantageously comprise the glycofurol in a proportion of about 1 to about 50%, preferably of about 5 to about 35%, 8 to 25%, 10 to 23%, 15 to 20% about 10%, 15%, 17%, 20%, 21%, 25% (percentages as weight by volume=W/V).

The co-solvent for the liquid carrier includes pharmaceutically acceptable solvents known in the formulation art.

According to the invention, the solvent is glycofurol; and the co-solvent is selected from the group consisting of acetone, acetonitrile, benzyl alcohol, butyl diglycol, dimethylacetamide, dimethylsulfoxide, dimethylformamide, N,N-diethyl-3-methylbenzamide (DEET), dipropylene glycol n-butyl ether, ethyl alcohol, isopropanol, methanol, phenylethyl alcohol, isopropanol, ethylene glycol monoethyl ether, ethylene glycol monomethyl ether, monomethylaceamide, dipropylene glycol monomethyl ether, liquid polyoxyethylene glycols, propylene glycol, N-methylpyrrolidone, 2-pyrrolidone, limonene, eucalyptol, diethylene glycol monoethyl ether, ethylene glycol, diethyl phthalate, polyethoxylated castor oil, methyl ethyl ketone, ethyl -L-lactate, and a mixture of at least two of these co-solvents.

In another embodiment the co-solvent is selected from the group consisting of dimethyl sulfoxide, acetone, dimethylacetamide, N,N-diethyl-3-methylbenzamide ethyl alcohol (DEET), dipropylene glycol monomethyl ether, methylethyl ketone, ethyl -L- lactate, and a mixture of at least two of these co-solvents.

In another embodiment the organic solvent in the local topical formulation is glycofurol and the organic co-solvent is selected from acetone, ethyl -L- lactate, dimethyl sulfoxide, dimethylacetamide and N,N-diethyl-3-methylbenzamide (DEET) and is preferably a mixture of at least two of acetone, ethyl -L- lactate, dimethyl sulfoxide, dimethylacetamide and N,N-diethyl-3-methylbenzamide (DEET).

The co-solvent can advantageously be present in the composition according to a volume/volume (V/V) ratio with respect to glycofurol of between about fraction 4/1 and about 1/4.

A pour-on or spot-on formulation generally can advantageously comprise acetone in a proportion of about 0 to about 50%, preferably of about 4 to about 35%, about 4%, 8%, 12%, , (percentages as volume by volume=V/V).

A pour-on or spot-on formulation generally can advantageously comprise dimethylacetamide in a proportion of about 0 to about 70%, preferably of about 5 to about 60%, about 32%, 35%, 36%, 38%,40%, 42%, 45%, 60% (percentages as volume by volume=V/V).

A pour-on or spot-on formulation generally can advantageously comprise dimethylsulfoxide in a proportion of about 0 to about 50%, preferably of about 10 to about 45%, about 35%, 40%, , (percentages as volume by volume=V/V).

A pour-on or spot-on formulation generally can advantageously comprise N,N-diethyl-3-methylbenzamide in a proportion of about 0 to about 30%, preferably of about 1 to about 25%, about 5%, 9%, 10%, 14%, 15%, 17%, (percentages as volume by volume=V/V).

The topical localized formulation can also include one or more additional ingredients. Examples of suitable additional ingredients are penetration enhancers, spreading agents, stabilizers such as antioxidants/preservatives, adhesion promoters and viscosity modifiers, crystallization inhibitors, UV blockers or absorbers, and colorants. Surface active agents, including anionic, cationic, non-ionic and ampholytic surface active agents, can also be included in these formulations.

In some embodiments, a topical formulation (pour-on or spot-on formulation) comprises a carrier that promotes the absorption or penetration of the isoxazoline through the skin into the blood stream, other bodily fluids (lymph), and/or body tissue (fat tissue). Contemplated examples of dermal penetration enhancers include, for example, dimethylsulfoxide, isopropyl myristate, dipropylene glycol pelargonate, silicone oil, aliphatic esters, triglycerides, and fatty alcohols.

Topical localized formulations also (or alternatively) may comprise, for example, one or more spreading agents. These substances act as carriers that assist in distributing an active ingredient over the animal recipient's coat or skin. They may include, for example, isopropyl myristate, dipropylene glycol pelargonate, silicone oils, fatty acid esters, triglycerides, and/or fatty alcohols.

Various spreading oil/solvent combinations also may be suitable, such as, for example, oily solutions, alcoholic and isopropanolic solutions (e.g., solutions of 2-octyl dodecanol or oleyl alcohol), solutions of esters of monocarboxylic acids (e.g., isopropyl myristate, isopropyl palmitate, lauric acid oxalic ester, oleic acid oleyl ester, oleic acid decyl ester, hexyl laurate, oleyl oleate, decyl oleate, and caproic acid esters of saturated fatty alcohols having a carbon chain of 12 to 18 carbons), solutions of esters of dicarboxylic acids (e.g., dibutyl phthalate, diisopropyl isophthalate, adipic acid diisopropyl ester, and di-n-butyl adipate), or solutions of esters of aliphatic acids (e.g., glycols). When the formulation comprises a spreading agent, it also may be advantageous to include a dispersant, such as, for example, pyrrolidin-2-one, N-alkylpyrrolidin-2-one, acetone, polyethylene glycol, or an ether or ester thereof, propylene glycol, or synthetic triglycerides.

Optionally a crystallization inhibitor can be present selected from the group consisting of an anionic surfactant, a cationic surfactant, a non-ionic surfactant, an amine salt, an amphoteric surfactant or polyvinylpyrrolidone, polyvinyl alcohols, copolymers of vinyl acetate and vinylpyrrolidone, polyethylene glycols, benzyl alcohol, mannitol, glycerol, sorbitol, polyoxyethylenated sorbitan esters; lecithin, sodium carboxymethylcellulose, and acrylic derivatives, or a mixture of these crystallization inhibitors.

The formulation can also comprise an antioxidizing agent intended to inhibit oxidation in air. Particularly preferred antioxidizing agents are those conventional in the art and include, for example, butylated hydroxyanisole, butylated hydroxytoluene, ascorbic acid, sodium metabisulphite, propyl gallate, sodium thiosulphate or a mixture of them.

Suitable exemplary polymers ("polymeric agents") for gelling and/or adhering that may be used in the compositions of the invention include, but are not limited to, colloidal silicone dioxide, ethyl cellulose, methyl cellulose, methacrylic esters copolymers, carboxylated vinyl acetate, and polyvinylpropylene (PVP)/Vinyl acetate copolymers, Poloxamer 124, Povidone K 17 Polysorbate 80 and Povidone K90.

The additional ingredients discussed above are well known to the practitioner in this art and may be obtained commercially or through known techniques.

The topical localized formulation is applied as a low volume of about 0.01 to 1 ml per kg, preferably about 0.05 to 0.1 ml per kg, with a total volume from 0.3 to 100 ml per animal, preferably limited to a maximum of about 50 ml depending on the target species.

For small companion animals such as dogs and cats the volume applied can be of the order of 0.3 to about 6 ml, preferably of the order of 0.4 to 2.0 ml per dose, for cats and of the order of 0.4 to about 5.0 ml for dogs, depending on the weight of the animal.

An exemplary composition for topical administration to warm-blooded animals typically comprises, on a weight to volume basis, about 15 to 20% w/v of glycofurol; about 5%-95% w/v of a co- solvent or solvent mixture, such as DMA by itself or in combination with about 10 to 20%w/v of acetone, and about 5 to 25% w/v of N,N-diethyl-3-methylbenzamide.

An exemplary composition for topical administration to warm-blooded animals typically comprises, on a weight to volume basis,about 5 to 25% w/v of glycofurol; about 5% to 95% v/v of a co- solvent or solvent mixture, such as N,N-diethyl-3-methylbenzamide by itself or in combination with about 10 to 50% v/v of DMA, and/ or about 10 to 20 % w/v of a cosolvent.

An exemplary composition for topical administration to warm-blooded animals typically comprises, on a weight to volume basis, about 5 to 25% w/v of glycofurol; about 5% to 95% v/v of a co- solvent or solvent mixture, such as DMA by itself or in combination with about 10 to 50%v/v of acetone, and/ or about 10 to 20 % v/v of a co-solvent.

In one embodiment of the invention the topical localized formulation comprise at least one isoxazoline compound of formula I and a macrocyclic lactone compound of the avermectin or milbemycin class of compounds. Macrocyclic lactone compounds are either natural products or are semi-synthetic derivatives thereof. The structure of at least certain macrocyclic lactone compounds are closely related, e.g., by sharing a complex 16-membered macrocyclic lactone ring.

One compound for use within the scope of the present invention is ivermectin. Another macrocyclic lactone is moxidectin. Moxidectin, also known as LL-F28249 alpha, is known from U.S. Patent No. 4,916,154. Another macrocyclic lactone is selamectin. Selamectin is 25-cyclohexyl-25-de(I- methylpropyl)-5 -deoxy-22,23 -dihydro-5 -(hydroxyimino)-avermectin B1 monosaccharide.

Another preferred compound is milbemycin, especially milbemycin oxime. Milbemycin, or B41, is a substance which is isolated from the fermentation broth of a milbemycin-producing strain of Streptomyces. The microorganism, the fermentation conditions and the isolation procedures are described in U.S. Patent Nos. 3,950,360 and 3,984,564.

Emamectin (4"-deoxy-4"-epi-methylaminoavermectin B1), which can be prepared as described in U.S. Patent Nos. 5,288,710 and 5,399,717, is a mixture of two homologues, 4"- deoxy-4"-epi-methylaminoavermectin Bla and 4"-deoxy-4"-epi-methylaminoavermectin B1. Preferably, a salt of emamectin is used.. Eprinomectin is chemically known as 4"-epi-acetylamino-4"-deoxy-avermectin B1.

For Latidectin, information can be found at "International Nonproprietary Names for Pharmaceutical Substances (INN)". World Health Organization (WHO) Drug Information, vol. 17, no. 4, page 278-279, (2003).

Lepimectin is (6R,13R,25R)-5-O-demethyl-28-deoxy-6,28-epoxy-13-[(Z)-[(methoxyimino)phenylacetyl]oxy]-25-methylmilbemycin B mixture with (6R,13R,25R)-5-O-demethyl-28-deoxy-6,28-epoxy-25-ethyl-13-[(Z)-[(methoxyimino)phenylacetyl]oxy]milbemycin B.

Most especially preferred are topical localized formulations, wherein the composition comprises) Compound A; and moxidectin; or Compound A; and selamectin, or Compound A; and milbemycin, or Compound A; and eprinomectin.

The macrocyclic lactone compounds are well known to a person skilled in the art and are easily obtained either commercially or through techniques known in the art.

The effective amount of the macrocyclic lactone compound is preferably between about 0.001 mg/ kg bodyweight, preferentially about 0.005 mg, and about 10 mg/kg. The proportions, by weight, of the isoxazoline compound and of the macrocyclic lactone compound are preferably between about 5/1 and about 1/0.0001.

Other biologically active compounds useful in the formulations of the present invention can be selected from Insect Growth Regulators (IGRs) such as e.g. fenoxycarb, lufenuron, diflubenzuron, novaluron, triflumuron, fluazuron, cyromazine, methoprene, pyriproxyfen etc., thereby providing both initial and sustained control of parasites (at all stages of insect development, including eggs) on the animal subject, as well as within the environment of the animal subject.

Most especially preferred are topical localized formulations, wherein the composition comprises Compound A and diflubenzuron or Compound A and methoprene, or Compound A and pyriproxyfen, or Compound A and fenoxycarb, or Compound A and fluazuron.

The effective amount of the IGR compound is preferably between about 0.1 mg/ kg bodyweight, preferentially about 1 mg, and about 10 mg. The proportions, by weight, of the isoxazoline compound and of the IGR compound are preferably between about 5/1 and about 0.000/1.

One aspect of the current disclosure is a method for permanently combating a parasite in an environment in which the animal is subjected to strong parasitic pressure where the administration of the topical localized formulation at a frequency far below a daily administration. The treatment according to the invention is carried out monthly, every 2 months, 3 months, 4 months, 5 months or 6 months on dogs or cats.

The time period between treatments depends upon factors such as the parasite(s) being treated, the degree of infestation, the type of mammal or bird and the environment where it resides. It is well within the skill level of the practitioner to determine a specific administration period for a particular situation.

In some embodiments of this invention, the topical localized formulation of the isoxazoline compound is administered to treat parasitoses of an animal (or make a medicament to treat parasitoses of an animal). The term "parasitoses" includes pathologic conditions and diseases associated with or caused by one or more ectoparasites directly, such as, for example, anemia and flea allergy dermatitis. It also includes pathologic conditions or diseases associated with caused by one or more vector-transmitted pathogens, such as, for example, Lyme disease, Ehrlichiosis (particularly canine ehrlichiosis), and Rocky Mountain spotted fever from vector ticks. The phrase "treatment of parasitoses" means to partially or completely inhibit the development of parasitoses of an animal susceptible to parasitoses, reduce or completely eliminate the symptoms of parasitoses of an animal having parasitoses, and/or partially or completely cure parasitoses of an animal having parasitoses. In general, the treatment of parasitoses is achieved by administering the formulation according to the invention comprising an isoxazoline to control an ectoparasite infestation.

This invention also relates to treatment methods wherein at least an ancillary goal of controlling ectoparasites in and/or on an animal is to control an ectoparasitic infestation in an environment that is occupied (periodically or continuously) by the animal. In some such embodiments, for example, the animal is a companion animal a cat or dog). The environment may be, for example, a house or other shelter; a room; a pen, a stall, or other confinement means; bedding; etc.

The topical localized formulations of the present invention are especially suitable for combating parasites that infest , canine (e.g., dogs), feline (e.g., house cats). Of particular note is the embodiment wherein the animals to be protected are domesticated dogs (i.e. Canis lupus familiaris) and domestic house cats (i.e. Felis catus).

In particular, the formulations of this invention are effective against ticks such as Ixodes spp., Boophilus spp., Rhipicephalus spp., Amblyomma spp., Dermacentor spp., Hyalomma spp. and Haemaphysalis spp.; and fleas such as Ctenocephalides felis (cat flea) and Ctenocephalides canis (dog flea).

This invention also is directed to kits that are, for example, suitable for use in performing the treatment methods described above. In general, such a kit will comprise a topical localized formulation according to the invention comprising a therapeutically effective amount of the isoxazoline compound, and an additional component(s). The additional component(s) may be, for example, one or more of the following: a diagnostic tool, instructions for administering the composition, an apparatus for administering the composition, a container comprising an excipient or other active ingredient to be mixed or administered in combination with the composition, or a memory aid (e.g., a stamp to adhere to a calendar to remind an animal owner of a time to administer a subsequent dose of the composition).

As used in the specification and claims, the terms "about" and "approximately" designate that a value is within a statistically meaningful range. Such a range can be typically within 20%, more typically still within 10%, and even more typically within 5% of a given value or range. The allowable variation encompassed by the terms "about" and "approximately" depends on the particular system under study, and can be readily appreciated by one of ordinary skill in the art.

As used herein, the term "w/w" designates weight/weight, the term "w/v" designates weight/volume, and the term "mg/kg" designates milligrams per kilogram of body weight.

Other advantages and characteristics of the invention will become apparent on reading the following description, given by way of non-limiting examples.

### EXAMPLE 1

### Preparation of Composition A

The calculated amount of e.g. 6.25 grams of 4-[5-(3,5-Dichlorophenyl)-5-trifluoromethyl-4,5-dihydroisoxazol-3-yl]-2-methyl-*N*-[(2,2,2-trifluoro-ethylcarbamoyl)-methyl]-benzamide (Compound A) were weighted and filled into a flask. The required volumes of excipients were added, e.g. 10 mL of DMA and 5 mL of glycofurol. The compound A was dissolved under mild stirring or shaking. This solution was brought to a final volume of 25 mL with acetone.

Using essentially the same procedure described hereinabove for composition A, composition B - K of table 2 and the formulations of table 3 were prepared. An alternative approach to the preparation was to weigh-in the excipients. The required weight was calculated based on the density of each product. Or, the order of addition was changed, e.g. excipients were blended and Compound A was introduced at a later stage.

Physicochemical parameters, that indicate the suitability of the formulations for topical localized (e.g. spot-on) administration, were evaluated. Compositions A to K of table 2 were tested using the following procedures
Viscosity: The newtonian viscosity (η) was determined by means of a rotational viscometer in a double gap cup and rotor system at 20 °C.
Evaporation: The evaporation was determined in a weight-recording balance placed in a fume cupboard. The sample pan was heated to 50 °C over 4 h and weight loss was recorded.
Spreading diameter: The spreading diameter was determined by measuring the diameter of three 20 µL spots of test product on a plastic sheet.
Water absorption: The water absorption was determined by determining the water concentration of a test product in contact with the surrounding atmosphere at a temperature of 25 °C after one day. In addition, the physical state of the test product, e.g. whether it was a clear solution, was also recorded.
Solubility: A saturated solution, i. e. a solution of a test compound in contact with undissolved particles of the test compound, was prepared and continuously shaken, temperature was recorded. The content of the compound in the solvent phase was determined by HPLC after approximately 24 h. The content result was taken as solubility. In some cases, the content was determined again after 48 h and the lower of the two results was taken as solubility.
Compatibility: Binary mixtures of the test compound and excipients were prepared and stored under defined storage conditions, e.g. 40 °C, 75 % RH. At study start and after defined storage periods, samples were analyzed for appearance, content and degradation products.

The physicochemical parameters of the formulations of Table 2 are summarized in Table 2a.

The results in Table 2a and the in vivo experiments where the formulations were administered to dogs show that the tested formulations are suitable for localized topical administration of isoxazoline compounds to animals.

### Example 2

### In vivo trials - spot-on administration of the formulations to dogs

The formulations of Table 2 were administered as spot-on to dogs at an 4-[5-(3,5-Dichlorophenyl)-5-trifluoromethyl-4,5-dihydroisoxazol-3-yl]-2-methyl-*N*-[(2,2,2-trifluoroethylcarbamoyl)-methyl]-benzamide (Compound A) dosage of 25 mg/ kg bodyweight. Dogs were observed for local and systemic tolerance of the treatment and the cosmetic appearance of the administration site was evaluated. Plasma samples were taken of all dogs pre-administration and 2, 4, 8 hours after administration, on Day, D1, D3, D7, D14 and subsequently weekly until D56. The plasma was analyzed for Compound A by HPLC-MS/MS.

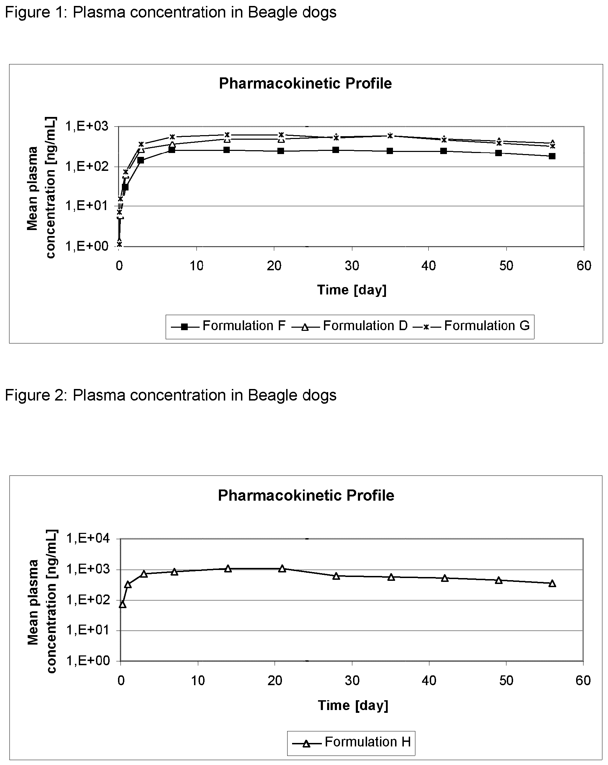

Results: The mean concentration of compound A in dog plasma is shown in Figures 1 and 2.

No local or systemic adverse reactions were observed. The cosmetic appearance was acceptable for the formulations, as only minor effects on appearance were detected for a short duration.

### Example 3

### In vivo trials - formulation comprising Compound A and moxidectin spot-on administration to dogs

Formulation N of Table 2 was administered as spot-on to dogs at an Compound A dosis of 25 mg/ kg bodyweight and moxidectin dosage of 2.5 mg/ kg bodyweight. Dogs were observed for local and systemic tolerance of the treatment and the cosmetic appearance of the administration site was evaluated. Plasma samples were taken of all dogs pre-administration 2, 4, 8 hours after administration, on Day 0, D1, D3, D7 and D14 and subsequently weekly until D56. The plasma was analyzed for Compound A and moxidectin concentration.

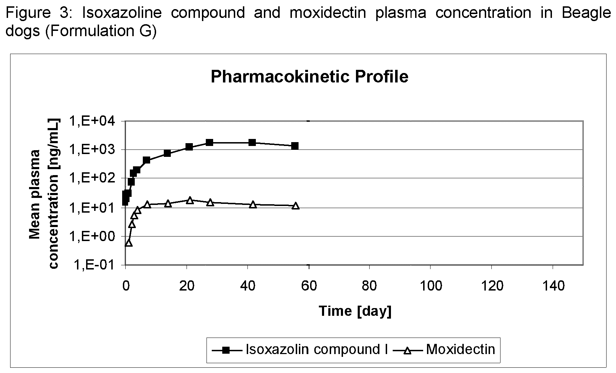

Results: The mean plasma concentration of the compound A and moxidectin in dogs is shown in Figure 3.

No local or systemic adverse reactions were observed. The cosmetic appearance was acceptable.

### Example 4

### Evaluation of the Efficacy of Test formulations against ticks in dogs

In this evaluation, Beagle dogs of mixed sex, were used and assigned to treatment and control groups .On day D -2, each dog was infested with 50 unfed adult ticks, R. sanguineus.

The dogs received on Day 0 .the treatments (formulations indicated in table 1) at a dosage of 25 mg/ kg body weight of the Compound A. The formulations were administered using a disposable pipette. The dose was applied as a line at the dorsal neck at the base of the skull.

One Group was left untreated. The dogs were observed for any immediate reactions to the treatments, and were observed for post-treatment adverse reactions, skin irritation, and behavior of test formulations at the time of treatment, after approximately 2, 4 and 8 hours, and on Days 1, 2 and 7 following administration of the treatments. Thereafter, dogs were observed once daily for the remainder of the study.

All ticks were removed 48 hours after treatment. Removed ticks were assessed according to the following categories: Efficacy no: for - live free, live attached- live engorged/ not engorged, and dead - engorged, Efficacy Yes: For dead, free, dead attached not engorged.

Tick counts were transformed and geometric means were used to calculate percent efficacy for the treatments. The results are shown in Table 1.

**Table 1: Result of in vivo efficacy studies**

| **Formulation No.** | | | **Study characteristics** | | | **Tick efficacy after 2 days [geometric mean, %]** | | | |
|---|---|---|---|---|---|---|---|---|---|
| D | | | R. sanguineus, 4 dogs, notional control groups | | | 86.0 - 93.3 | | | |
| F | | | R. sanguineus, 4 dogs, notional control groups | | | 94.8 - 97.5 | | | |
| G | | | R. sanguineus, 4 dogs, notional control groups | | | 96.5 - 98.3 | | | |
| H | | | R. sanguineus, 6 dogs, notional control groups | | | 91.8 - 96.1 | | | |
| E | | | I. ricinus, 6 cats, 30 mg/kg | | | 90.0 | | | |

| **Formulation No.** | **Active (mg)** | **Glycofurol (mL)** | **DEET (mL)** | **Acetone (mL)** | **DMSO (mL)** | **DMA (mL)** | **Ethyl Lactate (mL)** | **Limonene (mL)** | **Poloxamer 124 (mg)** |
|---|---|---|---|---|---|---|---|---|---|
| A | 500 | up to 1 ml | | | 0.35 | | | | |
| B | 280 | 0.20 | 0.11 | 0.41 | | 0.36 | | | |
| C | 500 | up to 1 ml | | | 0.35 | | | | 0.05 |
| D | 250 | 0.20 | | 0.22 | | 0.40 | | | |
| E | 280 | 0.17 | 0.14 | 0.14 | | 0.36 | | | |
| F | 250 | 0.20 | | 0.17 | | 0.40 | | 0.05 | |
| G | 250 | 0.20 | 0.10 | 0.18 | | 0.35 | | | |
| H | 280 | 0.20 | 0.10 | | | 0.35 | 0.16 | | |
| I | 280 | 0.20 | 0.10 | 0.16 | | 0.35 | | | |
| J | 280 | 0.17 | 0.14 | 0.10 | | 0.36 | 0.04 | | |
| K | 280 | 0.15 | 0.17 | 0.14 | | 0.35 | | | |

**Table 2a: Physicochemical parameters of formulations**

| **Formulation No.** | **Solubility [mg/mL]** | **Viscosity [mPas]** | **Evaporation [%]** | **Spreading [mm]** | **Water absorption after 1 d [appearance, %]** |
|---|---|---|---|---|---|
| A | | | | | |
| B | | 9.44 | 35.05 | | |
| C | | | | | |
| D | 620.4 | 3.87 | 48.63 | 17.9 | Turbid, 19.22 |
| E | | 8.63 | 34.91 | | |
| F | 591.0 | 4.20 | 43.35 | 19.04 | Turbid, 43.72 |
| G | 611.5 | 5.72 | 35.58 | 18.88 | Clear, 39.22 |
| H | 601.7 | 13.51 | 36.26 | 22.21 | Clear, 34.73 |
| I | 617.2 | 5.79 | 35.72 | 19.76 | Clear, 29.84 |
| J | | 8.09 | 32.82 | | |
| K | | 6.82 | 34.72 | | |

**Table 3: Formulations of Compound A Excipient: Amount [ml or mg] (utv= until total volume**

| active [mg] | 250 | 250 | 250 | 250 | 250 | 250 | 250 | 250 | 250 | 250 | 250 | 250 | 250 | 250 | 400 | 333 | 280 | 280 |
|---|---|---|---|---|---|---|---|---|---|---|---|---|---|---|---|---|---|---|
| Acetone [mL] | utv | utv | utv | utv | utv | utv | utv | utv | utv | utv | utv | utv | utv | | utv | utv | | 0.12 |
| DEET | | | | | | | | | | | | 0.05 | 0.1 | | | | 0.1 | 0.1 |
| Dimethylisosorbide | | | | | 0,05 | | | | | | | | | | | | | |
| DMA [mL] | 0.4 | 0.4 | 0.35 | 0.4 | 0.4 | 0.45 | 0.4 | 0.4 | 0.45 | 0.45 | 0.45 | 0.4 | 0.35 | 0.4 | 0.4 | 0.4 | 0.35 | 0.35 |
| DMSO [mL] | | | | | | | | | | | | | | | | | | |
| Ethyl L-lactate [mL] | | | | | | | | | | | | | | | | | utv | utv |
| Glycofurol [mL] | 0.2 | 0.25 | 0.25 | 0.2 | 0.2 | 0.25 | 0.15 | 0.15 | 0.2 | 0.15 | 0.2 | 0.2 | 0.2 | 0.2 | 0.2 | 0.2 | 0.2 | 0.2 |
| Limonene [mL] | | | | 0.05 | | | 0.15 | 0.05 | 0.05 | | | | | utv | | | | |

**Table 3 cont.: Formulations of Compound A Excipient: Amount [ml or mg] (utv= until total volume**

| active [mg] | 280 | 280 | 280 | 280 | 280 | 280 | 280 | 280 | 280 | 280 | 500 | 500 | 500 | 500 | 500 | 500 | 500 |
|---|---|---|---|---|---|---|---|---|---|---|---|---|---|---|---|---|---|
| Acetone [mL] | 0.08 | 0.04 | utv | utv | utv | utv | utv | utv | utv | utv | | | | | | | |
| DEET | 0.1 | 0.1 | 0.15 | 0.17 | 0.07 | 0.15 | 0.09 | 0.11 | 0.14 | 0.14 | | | | | | | |
| Dimethylisosorbide | | | | | | | | | | | | | | | | | |
| DMA [mL] | 0.35 | 0.35 | 0.42 | 0.35 | 0.4 | 0.32 | 0.38 | 0.36 | 0.36 | 0.6 | | | | | | | |
| DMSO [mL] | | | | | | | | | | | 0.35 | 0.35 | 0.35 | 0.35 | 0.35 | 0.4 | |
| Ethyl L-lactate [mL] | utv | utv | | | | | | | | 0.04 | | | | | | | |
| Glycofurol [mL] | 0.2 | 0.2 | 0.1 | 0.15 | 0.2 | 0.2 | 0.2 | 0.2 | 0.17 | 0.17 | utv | utv | utv | utv | utv | utv | utv |
| Limonene [mL] | | | | | | | | | | | | | | | | | |
| Moxidectin [mg] | | | | | | | | | | | | | | | | | |
| NMP [mL] | | | | | | | | | | | | | | | | | 0.35 |
| Poloxamer 124 [mL] | | | | | | | | | | | | | | 0.05 | | | |
| Polyethoxylated castor oil [mL] | | | | | | | | | | | | 0.05 | | | | | |
| Polysorbate 80 [mL] | | | | | | | | | | | | | 0.05 | | | | |
| Povidone K17 [mg] | | | | | | | | | | | | | | | 50 | | |
| Sorbitol [mg] | | | | | | | | | | | | | | | | 50 | |

| | | | | | | | | | | | | | | | | | |
|---|---|---|---|---|---|---|---|---|---|---|---|---|---|---|---|---|---|
| utv = up to 1 mL volume | | | | | | | | | | | | | | | | | |

## Claims

1. A topical localized spot-on or pour- on formulation for use in the treatment or prophylaxis of flea and tick infestation in dogs or cats which comprises an effective amount of at least one isoxazoline compound being 4-[5-(3,5-dichlorophenyl)-5-trifluoromethyl-4,5-dihydroisoxazol-3-yl]-2-methyl-*N*-[(2,2,2-trifluoro-ethylcarbamoyl)-methyl]-benzamide, and
a veterinary acceptable liquid carrier vehicle wherein the liquid carrier vehicle comprises glycofurol as a solvent, and a co-solvent selected from the group consisting of acetone, acetonitrile, benzyl alcohol, butyl diglycol, dimethylacetamide, dimethylsulfoxide, dimethylformamide, N,N-diethyl-3-methylbenzamide , dipropylene glycol n-butyl ether, ethyl alcohol, isopropanol, methanol, phenylethyl alcohol, isopropanol, ethylene glycol monoethyl ether, ethylene glycol monomethyl ether, monomethylaceamide, dipropylene glycol monomethyl ether, liquid polyoxyethylene glycols, propylene glycol, N-methylpyrrolidone, 2-pyrrolidone, limonene, eucalyptol, diethylene glycol monoethyl ether, ethylene glycol, diethyl phthalate, polyethoxylated castor oil, methyl ethyl ketone, ethyl-L-lactate, and a mixture of at least two of these co-solvents;
the formulation comprises 20 - 35% w/v of the isoxazoline compound in an application volume of 0.3 to 6 ml per animal; with long acting efficacy against ticks and fleas, so that the administration is carried out monthly, every 2 months, 3 months, 4 months, 5 months or 6 months.

2. The topical localized formulation for use according to claim 1, wherein the liquid carrier vehicle comprises glycofurol as solvent and a co-solvent selected from the group consisting of dimethyl sulfoxide, acetone, dimethylacetamide, N,N-diethyl-3-methylbenzamide, ethyl alcohol, eucalyptol, dipropylene glycol monomethyl ether, methylethyl ketone, ethyl-L-lactate, and a mixture of at least two of these co-solvents.

3. The topical localized formulation for use according to claim 2 wherein the liquid carrier vehicle comprises glycofurol as solvent and the organic co-solvent in the spot-on composition is a mixture of at least two of acetone, ethyl-L-lactate, dimethyl sulfoxide, dimethylacetamide and N,N-diethyl-3-methylbenzamide.

4. The topical localized formulation for use according to claims 1 to 3, wherein the composition comprises additionally an effective amount of a macrocyclic lactone compound selected from moxidectin, milbemycin oxime, selamectin, emamectin, latidectin and lepimectin or a salt thereof and/ or an insect growth regulator compound selected from fenoxycarb, lufenuron, diflubenzuron, novaluron, triflumuron, fluazuron, cyromazine, methoprene and pyriproxyfen.

5. The topical localized formulation for use according to claim 4, wherein the composition comprises an effective amount of 4-[5-(3,5-Dichlorophenyl)-5-trifluoromethyl-4,5-dihydroisoxazol-3-yl]-2-methyl-*N*-[(2,2,2-trifluoro-ethylcarbamoyl)-methyl]-benzamide and an effective amount of moxidectin.

6. The topical localized formulation for use according to claims 1 to 5, wherein an application volume of 0.4 to 2.0 ml of the topical localized formulation is administered to cats.

7. The topical localized formulation for use according to claims 1 to 5, wherein an application volume of 0.4 to 5.0 ml of the topical localized formulation is administered to dogs.

## Patentansprüche

1. Eine topische lokal begrenzte Spot-on- oder Pour-on-Formulierung zur Verwendung bei der Behandlung oder Prophylaxe von Floh- und Zeckenbefall bei Hunden oder Katzen, die eine wirksame Menge von mindestens einer Isoxazolin-Verbindung umfasst, nämlich 4-[5-(3,5-Dichlorphenyl)-5-trifluormethyl-4,5-dihydroisoxazol-3-yl]-2-methyl-N-[(2,2,2-trifluorethylcarbamoyl)-methyl]-benzamid, und ein veterinärmedizinisch akzeptables flüssiges Trägervehikel, wobei das flüssige Trägersystem Glycofurol als Lösungsmittel und ein Co-Lösungsmittel ausgewählt aus der Gruppe bestehend aus Aceton, Acetonitril, Benzylalkohol, Butyl-Diglykol, Dimethylacetamid, Dimethylsulfoxid, Dimethylformamid, N,N-Diethyl-3-methylbenzamid, Dipropylenglykol-n-Butylether, Ethanol, Isopropanol, Methanol, Phenylethylalkohol, Isopropanol, EthylenglykolMonoethylether, Ethylenglykol-Monomethylether, Monomethylacetamid, Dipropylenglykol-Monomethylether, flüssigen Polyoxyethylen-Glykolen, Propylenglykol, N-Methylpyrrolidon, 2-Pyrrolidon, Limonen, Eucalyptol, Diethylenglykol-Monoethylether, Ethylenglykol, Diethylphthalat, polyethoxylierte Rizinusöl, Methylethylketon, Ethyl-L-Lactat und einer Mischung aus mindestens zwei dieser Co-Lösungsmittel umfasst;
die Formulierung umfasst 20 - 35 % w/v der Isoxazolin-Verbindung in einem Anwendungsvolumen von 0,3 bis 6 ml pro Tier; mit langanhaltender Wirksamkeit gegen Zecken und Flöhe, sodass die Verabreichung monatlich, alle 2 Monate, 3 Monate, 4 Monate, 5 Monate oder 6 Monate erfolgt.

2. Die topische lokal begrenzte Formulierung zur Verwendung gemäß Anspruch 1, wobei das flüssige Trägervehikel Glycofurol als Lösungsmittel und ein Co-Lösungsmittel ausgewählt aus der Gruppe bestehend aus Dimethylsulfoxid, Aceton, Dimethylacetamid, N,N-Diethyl-3-methylbenzamid, Ethanol, Eucalyptol, Dipropylenglykol-Monomethylether, Methylethylketon, Ethyl-L-Lactat und einer Mischung aus mindestens zwei dieser Co-Lösungsmittel umfasst.

3. Die topische lokal begrenzte Formulierung zur Verwendung gemäß Anspruch 2, wobei das flüssige Trägersystem Glycofurol als Lösungsmittel umfasst und das organische Co-Lösungsmittel in der Spot-on-Zusammensetzung eine Mischung aus mindestens zwei von Aceton, Ethyl-L-Lactat, Dimethylsulfoxid, Dimethylacetamid und N,N-Diethyl-3-methylbenzamid ist.

4. Die topische lokal begrenzte Formulierung zur Verwendung gemäß den Ansprüchen 1 bis 3, wobei die Zusammensetzung zusätzlich eine wirksame Menge einer makrozyklischen Laktone-Verbindung umfasst, die aus Moxidectin, Milbemycin-Oxim, Selamectin, Emamectin, Latidectin und Lepimectin oder einem Salz davon ausgewählt ist und/oder eine Insektenwachstumsregulatorverbindung, die aus Fenoxycarb, Lufenuron, Diflubenzuron, Novaluron, Triflumuron, Fluazuron, Cyromazin, Methopren und Pyriproxyfen ausgewählt ist.

5. Die topische lokal begrenzte Formulierung zur Verwendung gemäß Anspruch 4, wobei die Zusammensetzung eine wirksame Menge von 4-[5-(3,5-Dichlorphenyl)-5-trifluormethyl-4,5-dihydroisoxazol-3-yl]-2-methyl-N-[(2,2,2-trifluorethylcarbamoyl)-methyl]-benzamid und eine wirksame Menge von Moxidectin umfasst.

6. Die topische lokal begrenzte Formulierung zur Verwendung gemäß den Ansprüchen 1 bis 5, wobei ein Anwendungsvolumen von 0,4 bis 2,0 ml der topischen lokal begrenzten Formulierung an Katzen verabreicht wird.

7. Die topische lokal begrenzte Formulierung zur Verwendung gemäß den Ansprüchen 1 bis 5, wobei ein Anwendungsvolumen von 0,4 bis 5,0 ml der topischen lokal begrenzten Formulierung an Hunde verabreicht wird.

## Revendications

1. Formulation localisée topique pour application locale ou à verser pour une utilisation dans le traitement ou la prophylaxie d'une infestation par des puces et des tiques chez des chiens ou des chats qui comprend une quantité efficace d'au moins un composé de type isoxazoline étant 4-[5-(3,5-dichlorophényl)-5-trifluorométhyl-4,5-dihydroisoxazol-3-yl]-2-méthyl-*N*-[(2,2,2-trifluoroéthylcarbamoyl)-méthyl]-benzamide, et un véhicule porteur liquide acceptable sur le plan vétérinaire, le véhicule porteur liquide comprenant du glycofurol en tant que solvant, et un cosolvant choisi dans le groupe constitué par l'acétone, l'acétonitrile, l'alcool benzylique, le butyl-diglycol, le diméthylacétamide, le diméthylsulfoxyde, le diméthylformamide, le N,N-diéthyl-3-méthylbenzamide, l'éther de n-butyle de dipropylèneglycol, l'alcool éthylique, l'isopropanol, le méthanol, l'alcool phényléthylique, l'isopropanol, le monoéthyléther d'éthylèneglycol, le monométhyléther d'éthylèneglycol, le monométhylacétamide, le monométhyléther de dipropylèneglycol, des polyoxyéthylèneglycols liquides, le propylèneglycol, la N-méthylpyrrolidone, la 2-pyrrolidone, le limonène, l'eucalyptol, le monoéthyléther de diéthylèneglycol, l'éthylèneglycol, le phtalate de diéthyle, l'huile de ricin polyéthoxylée, la méthyléthylcétone, le L-lactate d'éthyle, et un mélange d'au moins deux de ces cosolvants ;
la formulation comprenant 20 à 35 % p/v du composé de type isoxazoline dans un volume d'application de 0,3 à 0,6 ml par animal ; comportant une efficacité de longue durée contre des tiques et des puces, de sorte que l'administration est réalisée mensuellement, tous les 2 mois, tous les 3 mois, tous les 4 mois, tous les 5 mois ou tous les 6 mois.

2. Formulation localisée topique pour une utilisation selon la revendication 1, le véhicule porteur liquide comprenant du glycofurol en tant que solvant et un cosolvant choisi dans le groupe constitué par le diméthylsulfoxyde, l'acétone, le diméthylacétamide, le N,N-diéthyl-3-méthylbenzamide, l'alcool éthylique, l'eucalyptol, le monométhyléther de dipropylèneglycol, la méthyléthylcétone, le L-lactate d'éthyle, et un mélange d'au moins deux de ces cosolvants.

3. Formulation localisée topique pour une utilisation selon la revendication 2, le véhicule porteur liquide comprenant du glycofurol en tant que solvant et le cosolvant organique dans la composition pour application locale étant un mélange d'au moins deux parmi l'acétone, le L-lactate d'éthyle, le diméthylsulfoxyde, le diméthylacétamide et le N,N-diéthyl-3-méthylbenzamide.

4. Formulation localisée topique pour une utilisation selon les revendications 1 à 3, la composition comprenant de plus une quantité efficace d'un composé de type lactone macrocyclique choisi parmi la moxidectine, l'oxyde de milbémycine, la sélamectine, l'émamectine, la latidectine et la lépimectine ou un sel correspondant et/ou un composé régulateur de croissance d'insectes choisi parmi le fénoxycarbe, le lufénuron, le diflubenzuron, le novaluron, le triflumuron, le fluazuron, la cyromazine, le méthoprène et le pyriproxyfène.

5. Formulation localisée topique pour une utilisation selon la revendication 4, la composition comprenant une quantité efficace de 4-[5-(3,5-dichlorophényl)-5-trifluorométhyl-4,5-dihydroisoxazol-3-yl]-2-méthyl-*N*-[(2,2,2-trifluoroéthylcarbamoyl)-méthyl]-benzamide et une quantité efficace de moxidectine.

6. Formulation localisée topique pour une utilisation selon les revendications 1 à 5, un volume d'application de 0,4 à 2,0 ml de la formulation localisée topique étant administré à des chats.

7. Formulation localisée topique pour une utilisation selon les revendications 1 à 5, un volume d'application de 0,4 à 5,0 ml de la formulation localisée topique étant administré à des chiens.
